(19) Europäisches Patentamt
European Patent Office
Office européen des brevets

(11) Veröffentlichungsnummer: **0 174 565**
**B1**

(12) # EUROPÄISCHE PATENTSCHRIFT

(45) Veröffentlichungstag der Patentschrift: **25.10.89**

(51) Int. Cl.⁴: **C 07 D 211/46**, C 07 D 211/22, C 07 D 211/40, A 01 N 43/40

(21) Anmeldenummer: **85110928.0**

(22) Anmeldetag: **30.08.85**

(54) **Piperidinderivate und diese enthaltende Fungizide sowie Verfahren zur Bekämpfung von Pilzen.**

(30) Priorität: **08.09.84 DE 3433036**

(43) Veröffentlichungstag der Anmeldung: **19.03.86 Patentblatt 86/12**

(45) Bekanntmachung des Hinweises auf die Patenterteilung: **25.10.89 Patentblatt 89/43**

(84) Benannte Vertragsstaaten: **AT BE CH DE FR GB IT LI NL SE**

(56) Entgegenhaltungen: **EP-A-0 000 333**
**EP-A-0 008 686**

(73) Patentinhaber: **BASF Aktiengesellschaft, Carl-Bosch- Strasse 38, D-6700 Ludwigshafen (DE)**

(72) Erfinder: **Buschmann, Ernst, Dr., Georg- Ludwig-Krebs- Strasse 10, D-6700 Ludwigshafen (DE)**
Erfinder: **Schuster, Ludwig, Dr., Weinheimer Strasse 44, D-6703 Limburgerhof (DE)**
Erfinder: **Goetz, Norbert, Dr., Schoefferstrasse 25, D-6520 Worms 1 (DE)**
Erfinder: **Pommer, Ernst- Heinrich, Dr., Berliner Platz 7, D-6703 Limburgerhof (DE)**
Erfinder: **Ammermann, Eberhard, Dr., Sachsenstrasse 3, D-6700 Ludwigshafen (DE)**

EP 0 174 565 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft neue Piperidinderivate, Verfahren zu ihrer Herstellung, deren Verwendung als Fungizide, fungizide Mischungen, die diese Wirkstoffe enthalten, Verfahren zur Herstellung solcher fungizider Mischungen sowie Verfahren zur Bekämpfung von Schadpilzen mit diesen Wirkstoffen.

Es ist bekannt, Phenylpropylpiperidine, insbesondere das N-[3-(4-tertiär-Butylphenyl) -2-methyl-propyl]-4-hydroxipiperidin und die entsprechende -3-hydroximethylpiperidinverbindung als Fungizide zu verwenden (EP-A-333). Die Wirkung der bekannten Fungizide ist jedoch nicht in allen Anwendungsbereichen, insbesondere bei niedrigen Aufwandmengen und Konzentrationen, zufriedenstellend.

Es wurde gefunden, daß Piperidine der Formel

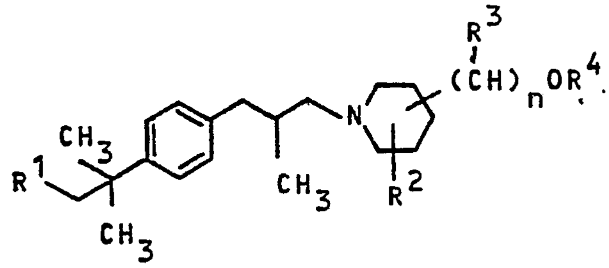

(1)

in der

$R^1$ Wasserstoff, Alkyl, Halogen

$R^2$, $R^3$ Wasserstoff, Alkyl

$R^4$ Wasserstoff, Alkyl, Alkenyl, Alkinyl, Halogenalkyl, Halogenalkenyl, gegebenenfalls durch Halogen, $C_1$-$C_4$-Alkyl oder Trifluormethyl substituiertes Benzyl oder alpha- oder beta- Naphthylmethyl, $COR^5$, $CO_2R^6$, $CONR^7R^8$

$R^5$ und $R^6$ Alkyl, gegebenenfalls durch Halogen oder $C_1$-$C_4$-Alkyl substituiertes Phenyl, gegebenenfalls durch Halogen substituiertes Benzyl

$R^7$ und $R^8$ Wasserstoff, Alkyl, gegebenenfalls durch Halogen substituiertes Phenyl, gegebenenfalls durch Halogen substituiertes Benzyl und

n die Zahlen 0, 1, 2 und 3 bedeuten, und ihre für Pflanzen verträglichen Säureadditionssalze, mit der Maßgabe, daß nicht gleichzeitig $R^4$ Wasserstoff, Acetyl oder Propionyl, n 0 oder 1 und $R^1$, $R^2$ und $R^3$ Wasserstoff bedeuten,

eine sehr gute fungizide Wirkung haben, die der Wirkung der bekannten Piperidine überlegen ist.

$R^1$ bedeutet beispielsweise Wasserstoff, $C_1$-$C_6$-Alkyl, Methyl, Ethyl, Propyl, n-Butyl, Isopropyl, Isobutyl, n-Pentyl, n-Hexyl, Chlor, Brom, Fluor.

$R^2$ und $R^3$ bedeuten unabhängig voneinander beispielsweise: Wasserstoff, $C_1$-$C_6$-Alkyl, Methyl, Ethyl, Propyl, Isopropyl, n-Butyl, iso-Butyl, n-Pentyl, n-Hexyl.

$R^4$ bedeutet beispielsweise Wasserstoff, $C_1$-$C_6$-Alkyl, Methyl, Ethyl, Propyl, Isopropyl, n-Butyl, iso-Butyl, n-Pentyl, n-Hexyl, Allyl, Propargyl, Trichlorallyl, ($CH_2CCl = CCl_2$), Benzyl, Halogenbenzyl, 4-Chlorbenzyl, Dihalogenbenzyl, 2,4-Dichlorbenzyl, $C_1$-$C_4$-Alkylbenzyl, 4-Methylbenzyl, 4-tert.-Butyl-benzyl, Trifluormethylbenzyl, 4-$CF_3$-Benzyl, alpha- oder beta-Naphthylmethyl.

$R^5$ und $R^6$ bedeuten unabhängig voneinander beispielsweise $C_1$-$C_4$-Alkyl, Methyl, Ethyl, n-Propyl, iso-Propyl, n-Butyl, Phenyl, Halogenphenyl, 4-Chlorphenyl, Dihalogenphenyl, 3,5-Dichlorphenyl, 2,4-Dichlorphenyl, $C_1$-$C_4$-Alkylphenyl, 4-Methylphenyl, 3-Chlorphenyl, Benzyl, Halogenbenzyl, 4-Chlorbenzyl.

$R^7$ und $R^8$ bedeuten unabhängig voneinander beispielsweise Wasserstoff, $C_1$-$C_4$-Alkyl, Methyl, Ethyl, n-Propyl, iso-Propyl, Phenyl, Halogenphenyl, 4-Chlorphenyl, 3-Chlorphenyl, Dihalogenphenyl, 3,5-Dichlorphenyl, Benzyl, Halogenbenzyl, 4-Chlorbenzyl. Säureadditionssalze sind z. B. die Salze mit anorganischen oder organischen Säuren, beispielsweise Salzsäure, Bromwasserstoffsäure, Schwefelsäure, Phosphorsäure, Essigsäure, Propionsäure, Dodecylbenzolsulfonsäure.

Die neuen Verbindungen können als Fungizide Verwendung finden.

Die neuen Verbindungen können beispielsweise hergestellt werden, indem man ein Phenylalkylhalogenid der Formel

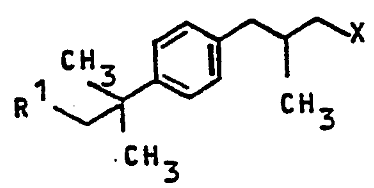

(2)

in der $R^1$ die oben angegebene Bedeutung hat und X für Chlor oder Brom steht, oder einen entsprechenden Aldehyd der Formel

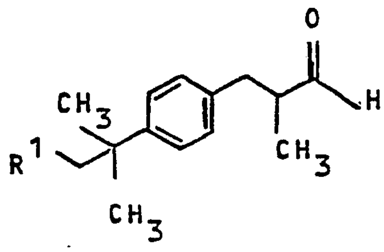

(3)

in der $R^1$ die oben angegebenen Bedeutungen hat, umsetzt mit einen Piperidin der Formel

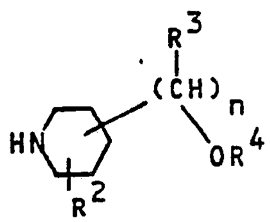

(4)

in der $R^2$, $R^3$, $R^4$ und n die oben genannten Bedeutungen haben, wobei die Umsetzung des Aldehyds unter reduzierenden Bedingungen erfolgt.

Sie können ferner beispielsweise hergestellt werden, indem man ein Hydroxypiperidin der Formel

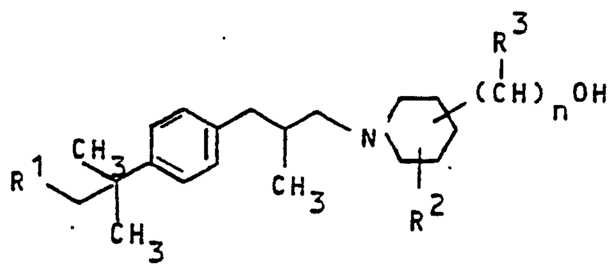

(5)

in der $R^1$, $R^2$, $R^3$ und n die oben genannten Bedeutungen haben,

mit einem Alkylierungsmittel der Formel

$R^{4'}Y$ (6)

in der $R^{4'}$ Alkyl, Alkenyl, Alkinyl, Halogenalkyl, Halogenalkenyl oder gegebenenfalls durch Halogen, $C_1$-$C_4$-Alkyl oder Trifluormethyl substituiertes Benzyl oder alpha- oder beta-Naphthylmethyl und Y Cl, Br, J bedeuten, oder

mit einem Acylchlorid der Formeln

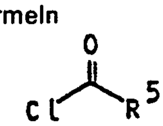

(7)

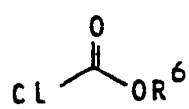

(8)

oder mit einem Isocyanat oder Carbamoylchlorid der Formel

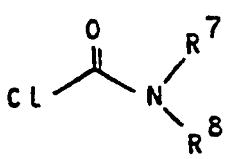

(9)

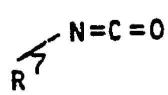

(10) .

in denen $R^5$, $R^6$, $R^7$ und $R^8$ die oben genannten Bedeutungen außer Wasserstoff haben, umsetzt.

Wichtige Ausgansverbindungen für die Herstellung der Piperidine sind die Phenylalkylhalogenide der Formel (2) und die entsprechenden Aldehyde der Formel (3),

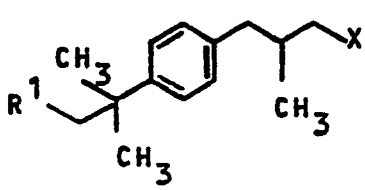

(2)

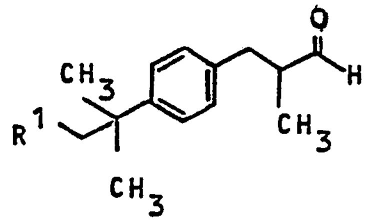

(3)

die in EP-9 077 und DE-OS-2 752 036 beschrieben sind. Die Synthese der neuen Verbindungen kann beispielsweise erfolgen wie im folgenden Schema gezeigt, wobei die einzelnen Reste die oben genannten Bedeutungen haben und X Chlor oder Brom bedeutet.

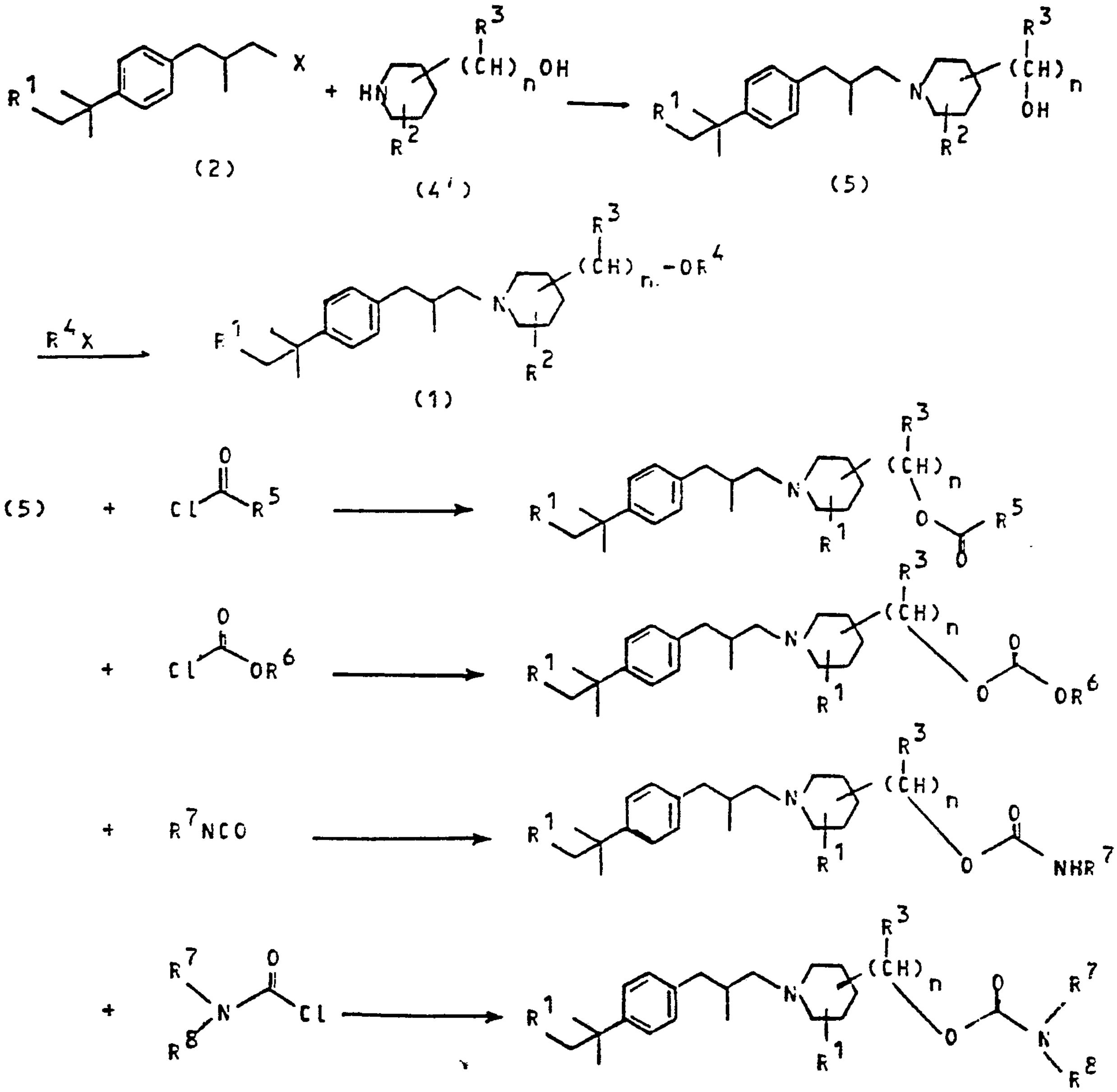


(2) (4') (5)

(1)

(5)

Als Amine der Formel 4'

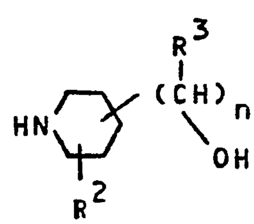

(4')

kommen beispielsweise in Frage:

3-Hydroxypiperidin
4-Hydroxypiperidin
4-Hydroxymethylpiperidin
3-Hydroxymethylpiperidin
4-Hydroxyethylpiperidin
3-(1-Hydroxyethyl)-piperidin
3-(1-Hydroxypropyl)-piperidin
3-(1-Hydroxybutyl)-piperidin
3-(1-Hydroxypentyl)-piperidin
3-(1-Hydroxyhexyl )-piperidin
3-(1-Hydroxy-isobutyl)-piperidin
3-(1-Hydroxy-isopentyl)-piperidin
3-(1-Hydroxybutyl)-4-propyl-piperidin
3-(1-Hydroxypentyl)-4-butyl-piperidin
3-(1-Hydroxyhexyl)-4-pentyl-piperidin

Die oben aufgeführten Piperidine sind in bekannter Weise durch Hydrierung entsprechender Hydroxy- oder Acylpyridine zugänglich. Die Synthese von Acylpyridinen wird beschrieben in DE-OS-3 126 819.

Die folgenden Vorschriften und Beispiele erläutern die Herstellung der neuen Verbindungen und ihrer Vorprodukte.

**Vorschrift 1**

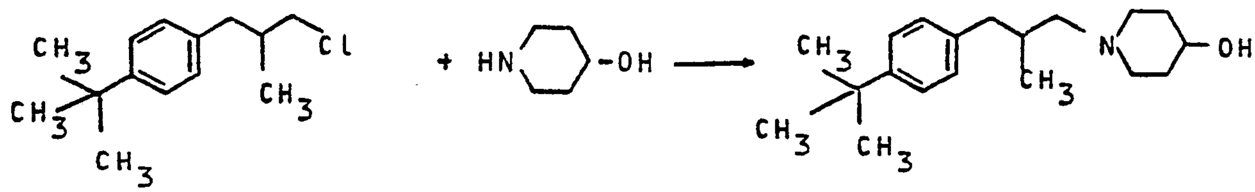

Zu einer Lösung von 112 g 3-(4-tert.-Butylphenyl)-2-methyl-propylchlorid und 50 g 4-Hydroxypiperidin in 1 Liter Dimethylformamid (DMF) werden 106 g $Na_2CO_3$ zugegeben. Man erwärmt das Ganze 15 Stunden auf 150°C, engt ein und nimmt den Rückstand in $CH_2Cl_2$ auf. Die $CH_2Cl_2$-Phase wird mit Wasser gewaschen, über $Na_2SO_4$ getrocknet und eingeengt. Destillation des Rohproduktes ergibt 103 g N-[3-(4-tert.-Butylphenyl)-2-methyl-propyl]-4-hydroxypiperidin [A] als leicht gelbliches Öl.

Sdp. 175 bis 178° C/0,4 mbar.

[A] ist eine Vorstufe für die Herstellung der Verbindungen Nr. 49, 51, 52, 53, 54, 55.

**Vorschrift 2**

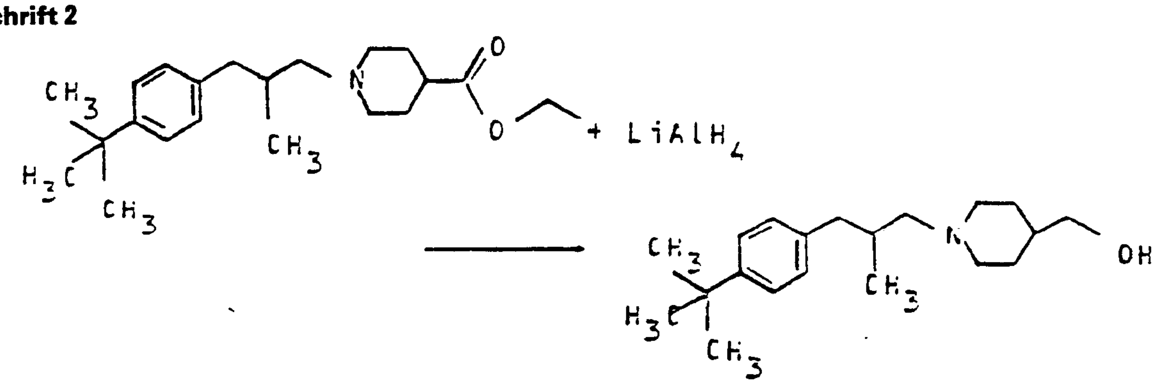

Zu einer Suspension von 10 g $LiAlH_4$ in 100 ml abs. Ether wird zugetropft eine Lösung von 41,4 g N-[3-(4-tert.-Butylphenyl)-2-methyl-propyl]-4-carbethoxypiperidin in 100 ml abs. Diethylether (exotherme Reaktion, Ether erwärmt sich zum Rückfluß). Nach Abklingen der Reaktion wird 4 h zum Rückfluß erwärmt. Unter Eiskühlung werden langsam 100 ml Wasser zugetropft. Man gibt 500 Diethylether zu, dekantiert ab und verreibt den schmierigen Rückstand noch zweimal mit Ether. Die vereinigten Etherphasen werden mit Wasser gewaschen, über $Na_2SO_4$ getrocknet und eingeengt. Destillation des Rückstandes ergibt 21 g N-[3-(4-tert.-Butylphenyl)-2-methyl-propyl]-4-hydroxymethylpiperidin [B].

Sdp. 175 - 178°C/0,2 mbar.

[B] ist eine Vorstufe für die Herstellung der Verbindungen Nr. 56, 57.

**Herstellungsbeispiel 1**

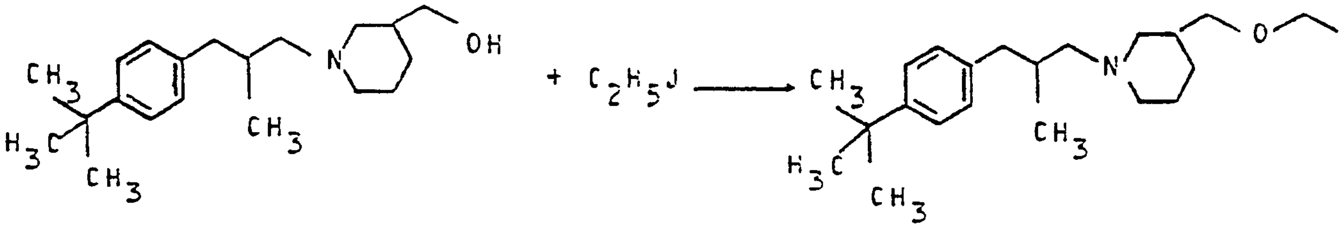

Zu einer Lösung von 60,6 g N-[3-(4-tert.-Butyl)-phenyl-2-methyl-propyl]-3-hydroxymethylpiperidin in 110 ml Dimethylsulfoxid (DMSO) und 390 ml Diethylether (Ether) werden portionsweise 13,5 g 80-%-ige NaH-Suspension in Paraffin zugegeben. Man erwärmt 1 h zum Rückfluß, tropft 62,4 g Ethyljodid zu und rührt 30 min. nach. Nach vorsichtiger Hydrolyse mit 1,2 l $H_2O$ (Wasser zunächst langsam zutropfen) wird mit Ether extrahiert, mit wäßriger Natriumthiosulfatlösung und mit Wasser gewaschen, über $Na_2SO_4$ getrocknet und eingeengt. Gaschromatographische Untersuchung des Rohproduktes zeigte nur 80-%-ige Umsetzung. Das Rohprodukt wird deshalb nochmals mit den gleichen Mengen NaH und $C_2H_5J$ umgesetzt. Die Aufarbeitung erfolgte wie oben. Destillation ergab 58 g leicht gelbliches Öl.

Sdp. 160 - 164° C/0,4 mbar

(Verbindung Nr. 13).

**Herstellungsbeispiel 2**

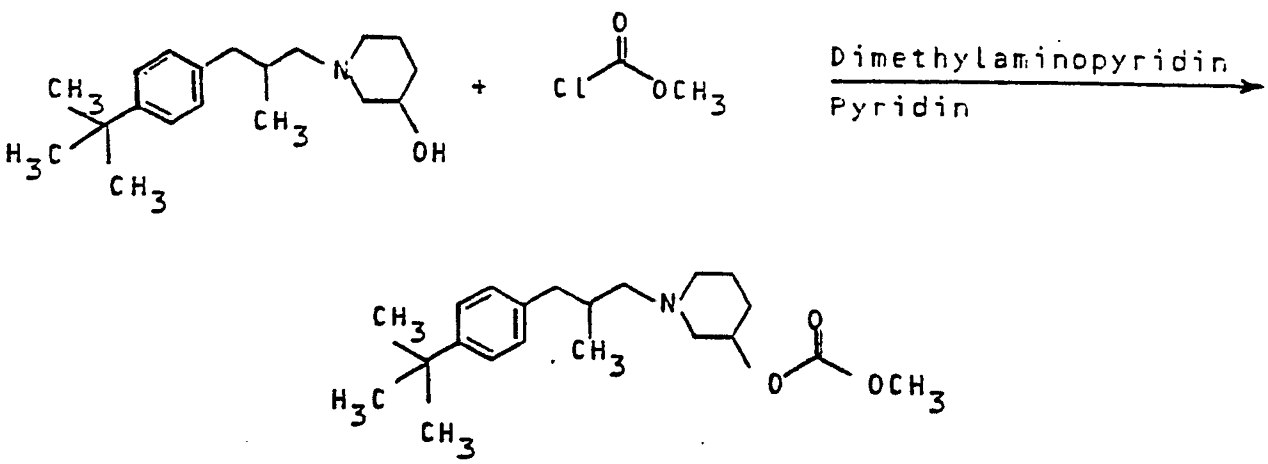


Zu einer Lösung von 40 g N-[3-(4-tert.-Butylphenyl-2-methyl-propyl]-3-hydroxypiperidin, 132 g Pyridin, 0,2 g 4-Dimethylaminopyridin in 500 ml Tetrahydrofuran (THF) werden 16 g Chlorameisensäuremethylester zugetropft. Man rührt 15 h bei Raumtemperatur, engt ein, nimmt den Rückstand mit $CH_2Cl_2$ auf, wäscht mit Wasser, verd. NaOH-Lösung und wieder Wasser, trocknet über $Na_2SO_4$ engt ein und destilliert. Ausbeute 16 g N-[3-(4-tert.-Butylphenyl-2-methyl-propyl]-3-oxy-carbomethoxy-piperidin.

Sdp. 180 bis 182° C/0,3 mbar.

(Verbindung Nr. 44).

Auf entsprechende Weise können die in folgenden Tabellen aufgeführten Piperidine hergestellt werden.

**Tabelle 1**

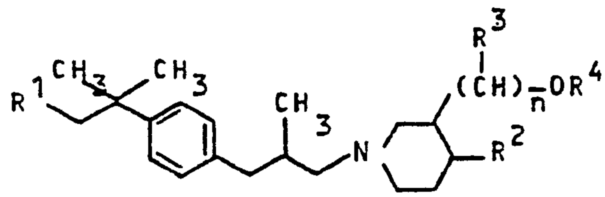

| Verbindung Nr | $R^1$ | $R^2$ | $R^3$ | $R^4$ | n | Spd./mbar |
|---|---|---|---|---|---|---|
| 1 | H | H | - | $CH_3$ | 0 | 145 - 152° C/0,4 |
| 2 | $CH_3$ | H | - | $CH_3$ | 0 | 156 - 162° C/0,4 |
| 3 | Cl | H | - | $CH_3$ | 0 | |
| 4 | H | H | - | $C_2H_5$ | 0 | 145 - 153° C/0,4 |
| 5 | $CH_3$ | H | - | $C_2H_5$ | 0 | |
| 6 | Cl | H | - | $C_2H_5$ | 0 | |
| 7 | H | H | - | n-$C_3H_7$ | 0 | |
| 8 | H | H | - | Allyl | 0 | 163 - 168° C/0,4 |
| 9 | $CH_3$ | H | - | Allyl | 0 | |
| 10 | H | H | - | Benzyl | 0 | 202 - 204° C/0,4 |
| 11 | $CH_3$ | H | - | Benzyl | 0 | |
| 12 | H | H | - | 4-Cl-Benzyl | 0 | |
| 13 | H | H | H | $CH_3$ | 1 | 160 -164° C/0,4 |
| 14 | $CH_3$ | H | H | $CH_3$ | 1 | |
| 15 | H | H | H | $C_2H_5$ | 1 | 147 - 154° C/0,3 |
| 16 | H | H | H | n-Propyl | 1 | |
| 17 | H | H | H | iso-Propyl | 1 | |
| 18 | H | H | H | Allyl | 1 | 170 - 182° C/0,5 |
| 19 | $CH_3$ | H | H | Allyl | 1 | |
| 20 | H | H | H | Benzyl | 1 | 226 - 230° C/0,9 |
| 21 | H | H | H | 2,4-$Cl_2$-Benzyl | 1 | 224 - 225° C/0,3 |
| 22 | H | H | H | 4-$CH_3$-Benzyl | 1 | |
| 23 | H | H | H | 3-$CH_3$-Benzyl | 1 | |
| 24 | H | H | $CH_3$ | H | 1 | 180 - 186° C/0,9 |
| 25 | H | H | $CH_3$ | $CH_3$ | 1 | 153 - 162° C/0,4 |
| 26 | H | H | $CH_3$ | Allyl | 1 | |
| 27 | H | H | $CH_3$ | Benzyl | 1 | |
| 28 | $CH_3$ | H | $CH_3$ | H | 1 | |
| 29 | $CH_3$ | H | $CH_3$ | $CH_3$ | 1 | |
| 30 | $CH_3$ | H | $CH_3$ | $C_2H_5$ | 1 | |
| 31 | H | n-Propyl | n-Propyl | H | 1 | |
| 32 | H | n-Propyl | n-Propyl | $CH_3$ | 1 | |
| 33 | H | n-Butyl | n-Butyl | H | 1 | |
| 34 | H | n-Butyl | n-Butyl | $CH_3$ | 1 | |
| 35 | H | n-Pentyl | n-Pentyl | H | 1 | |
| 36 | H | n-Pentyl | n-Pentyl | $CH_3$ | 1 | |
| 37 | H | H | n-Propyl | H | 1 | |
| 38 | H | H | n-Propyl | $CH_3$ | 1 | |
| 39 | H | H | n-Propyl | Allyl | 1 | |
| 40 | H | H | n-Butyl | H | 1 | |
| 41 | H | H | n-Butyl | $CH_3$ | 1 | |
| 42 | H | H | n-Pentyl | H | 1 | 180 - 182° C/0,3 |
| 43 | H | H | n-Pentyl | $CH_3$ | 1 | |
| 44 | H | H | - | $CO_2CH_3$ | 0 | 180 - 182° C/0,3 |
| 45 | H | H | - | $CONHCH_3$ | 0 | |
| 46 | H | H | H | $CO_2O_2H_5$ | 1 | 180° C/0,3 |
| 47 | H | H | H | Propargyl | 1 | 168 - 172° C/0,3 |
| 48 | H | H | H | $CH_2CCl=CCl_2$ | 1 | |
| 66 | H | H | $CH_3$ | $COCH_3$ | 1 | 165° C/0,3 |
| 67 | H | H | - | $CO_2C_2H_5$ | 0 | 182° C/0,3 |
| 68 | H | H | H | $CO_2CH_3$ | 1 | 184 - 187° C/0,3 |

**Tabelle 2**

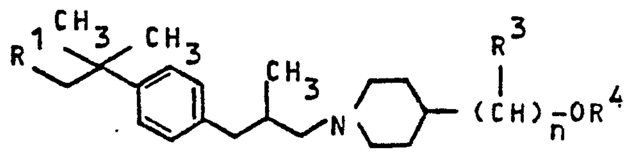

| Verbindung Nr. | $R^1$ | $R^3$ | $R^4$ | n | Spd./mbar |
|---|---|---|---|---|---|
| 49 | H | - | $CH_3$ | 0 | 152 - 156°C/0,4 |
| 50 | $CH_3$ | - | $CH_3$ | 0 | |
| 51 | H | - | $C_2H_5$ | 0 | 156 - 160°C/0,4 |
| 52 | Cl | - | $C_2H_5$ | 0 | |
| 53 | H | - | Allyl | 0 | |
| 54 | H | - | Benzyl | 0 | |
| 55 | H | - | $CO_2CH_3$ | 0 | |
| 56 | H | H | $CH_3$ | 1 | |
| 57 | H | H | $C_2H_5$ | 1 | |
| 58 | H | $CH_3$ | H | 1 | |
| 59 | H | $CH_3$ | $CH_3$ | 1 | |
| 60 | H | H | H | 2 | 186 - 190°C/0,4 |
| 61 | $CH_3$ | H | H | 2 | |
| 62 | H | H | $CH_3$ | 2 | 169 - 175°C/0,4 |
| 63 | H | H | Allyl | 2 | 185 - 190°C/0,4 |
| 64 | H | H | Acetyl | 2 | |
| 65 | H | H | $CO_2CH_3$ | 2 | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, Weinbau sowie Gemüse - wie Gurken, Bohnen und Kürbisgewächse.

Die neuen Verbindungen sind insbesondere geeignet zur Bekämpfung folgender Pflanzenkrankheiten:

Erysiphe graminis (echte Mehltau) in Getreide,
Erysiphe cichoracearum an Kürbisgewächsen,
Podosphaera leucotriche an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Pseudocercosporella herpotrichoides an Weizen, Gerste,
Pyricularia oryzae an Reis,
Alternaria solani an Kartoffeln, Tomaten,
Verticillum-Arten in Baumwolle und Gemüse,
Plasmopara viticola an Reben.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen: Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Ketone (z. B. Cyclohexanon), Amine (z. B. Ethanolamin, Dimethylformamid) und Wasser; Trägerstoffe wie natürliche Gesteinsmehle, (z. B. Kaoline, Tonerden, Talkum, Kreide) und synthetische Gesteinsmehle (z. B. hochdisperse Kieselsäure, Silikate); Emulgiermittel wie nichtionogene und anionische Emulgatoren (z. B. Polyoxyethylen-Fettalkohol-Ether, Alkylsulfonate und Arylsulfonate) und Dispergiermittel wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liege je nach Art des gewünscht er Effektes zwischen 0,02 und 3 kg Wirkstoff oder mehr je ha. Die neuen Verbindungen können auch im Materialschutz u.a. zur Bekämpfung holzzerstörender Pilze wie Coniophora puteana und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung Nr. 1 mit 10 Gewichtsteilen N-Methyl-alpha-pyrrolidon und erhält eine Lösung, die zur Anwendung in Form kleinster Tropfen geeignet ist.

II. 20 Gewichtsteile der Verbindung Nr. 4 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung Nr. 13 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gewichtsteile der Verbindung Nr. 49 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes vor 40 Mol Ethylenoxid an 1 Mol Ricinusöl besteht. Durch Eingießen und feines Verteilen der Lösung in Wasser erhält man eine wäßrige Dispersion.

V. 80 Gewichtsteile der Verbindung Nr. 51 werden den mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

VI. 3 Gewichtsteile der Verbindung Nr. 62 werden mit 97 Gewichtsteilen feinteiligem Kaolin innig vermischt. Man erhält auf diese Weise ein Stäubemittel, das 3 Gew.-% des Wirkstoffs enthält.

VII. 30 Gewichtsteile der Verbindung Nr. 63 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung Nr. 4 werden mit 10 Teilen Natriumsalz eines Phenolsulfonsäureharnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung Nr. 13 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z. B. Herbiziden, Insektiziden, Wachstumsregulatoren und Fungiziden oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten beispielsweise erläutern.

Fungizide, die mit den neuen Verbindungen kombiniert werden können, sind beispielsweise:

Schwefel,

Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,

heterocyclische Strukturen, wie
2-Heptadecyl-2-imidazolin-acetat
2,4-Dichlor-6-(o-chloranilino)-s-triazin
0,0-Diethyl-phthalimidophosphonothioat
5-Amino-1-(bis-(dimethylamino )-phosphinyl)-3-phenyl-1,2,4-triazol
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
2-Methoxycarbonylamino-benzimidazol
2-(Furyl-2)-benzimidazol
2-(Thiazolyl-4)-benzimidazol
N-(1,1,2,2-Tetrachlorethylthio)-tetrahydrophthalimid
N-Trichlormethylthio-tetrahydrophthalimid
N-Trichlormethylthio-phthalimid

N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Iod-benzoesäure-anilid
N-Formyl-N-morpholin-2,2,2-trichlorethylacetal
Piperazin-1,4-diylbis-1-(2,2-2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1-H-1,2,4-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
alpha-(2-Chlorphenyl)-alpha-(4-chlorphenyl)5-pyrimidin-methanol
5-Butyl-2-dimethylamino-4-hydroxy-6-methyl-pyrimidin
Bis-(p-chlorphenyl)-3-pyridinmethanol

1,2-Bis-(3-ethoxycarbonyl-2-thioureido)-benzol
1,2-Bis-(3-methoxycarbonyl-2-thioureido)-benzol

sowie verschiedene Fungizide, wie
Dodecylguanidinacetat
3-(3-(3,5-Dimethyl-2-oxycyclohexyl)-2-hydroxyethyl)-gluataramid
Hexachlorbenzol
DL-Methyl-N-(2,6-dimethyl-phenyl)-N-furoyl-(2)-alaninat,
DL-N-(2,6-Dimethyl-phenyl)-N-(2'-methoxyacetyl)-alaninmethylester
N-(2,6-Dimethylphenyl)-N-chloracetyl-DL-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl)-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid
2-Cyano-N-(ethylaminocarbonyl)-2-methoximino-acetamid
1-[2-(2,4-Dichlorphenyl)-pentyl]-1H-1,2,4-triazol
4-Difluor-alpha-(1H-1,2,4-triazolyl-1-methyl)-benzhydrylalkohol

Für die folgenden Versuche wurden als Vergleichsmittel die aus EP-A-333 bekannten Verbindungen N-[3-(4-tertiär-Butylphenyl)-2-methyl-propyl]-4-hydroxipiperidin (B) und N-[3-(4-tertiär-Butylphenyl)-2-methyl-propyl]-3-hydroximethylpiperidin (A) verwendet.

## Versuch 1

Wirksamkeit gegen Weizenmehltau

Blätter von in Töpfen gewachsenen Weizenkeimlingen der Sorte "Frühgold" werden mit wäßriger Spritzbrühe, die 80 % Wirkstoff und 20 % Emulgiermittel in der Trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit aufgestellt. Nach 7 Tagen wird das Ausmaß der Mehltauentwicklung ermittelt.

Das Ergebnis des Versuches zeigt, daß bei der Anwendung als 0,025-%-ige und 0,006-%-ige Spritzbrühen die Verbindungen 1, 4, 13, 49, 51, 62 und 63 eine bessere fungizide Wirkung zeigten (97 %) als der bekannte Wirkstoff A (90 %).

## Versuch 2

Wirksamkeit gegen Plasmopara viticola

Blätter von Topfreben der Sorte "Müller-Thurgau" werden mit wäßriger Spritzbrühe, die 80 % Wirkstoff und 20 % Emulgiermittel in der Trockensubstanz enthält, besprüht. Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von Plasmopara viticola (Rebenperonospora) infiziert. Danach werden die Reben zunächst für 16 Stunden in einer wasserdampfgesättigten Kammer bei 24°C und anschließend für 8 Tage in einem Gewächshaus mit Temperaturen zwischen 20 und 30°C aufgestellt. Nach dieser Zeit werden die Pflanzen zur Beschleunigung des Sporangienträgerausbruches abermals für 16 Stunden in der feuchten Kammer aufgestellt. Dann erfolgt die Beurteilung des Ausmaßes des Pilzausbruches auf den Blattunterseiten.

Das Ergebnis des Versuches zeigt, daß bei der Anwendung als 0,05-%-ige Spritzbrühe die Verbindungen 8, 13, 49, 51 und 63 eine bessere fungizide Wirkung zeigten (97 %) als die bekannten Wirkstoffe A und B (70 %).

## Patentansprüche

1. Piperidin der Formel

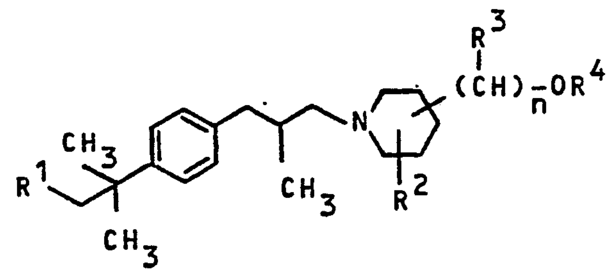

(1) .

in der
$R^1$ Wasserstoff, Alkyl, Halogen,
$R^2$, $R^3$ Wasserstoff, Alkyl,
$R^4$ Wasserstoff, Alkyl, Alkenyl, Alkinyl, Halogenalkyl, Halogenalkenyl, gegebenenfalls durch Halogen, $C_1$-$C_4$-Alkyl oder Trifluormethyl substituiertes Benzyl oder alpha- oder beta-Naphthylmethyl, $COR^5$, $CO_2R^6$, $CONR^7R^8$,
$R^5$ und $R^6$ Alkyl, gegebenenfalls durch Halogen oder $C_1$-$C_4$-Alkyl substituiertes Phenyl, gegebenenfalls durch Halogen substituiertes Benzyl,
$R^7$ und $R^8$ Wasserstoff, Alkyl, gegebenenfalls durch Halogen substituiertes Phenyl, gegebenenfalls durch Halogen substituiertes Benzyl, und
n die Zahlen 0, 1, 2 und 3 bedeuten, und seine für Pflanzen verträglichen Säureadditionssalze mit der Maßgabe, daß nicht gleichzeitig $R^4$ Wasserstoff, Acetyl oder Propionyl, n 0 oder 1 und $R^1$, $R^2$ und $R^3$ Wasserstoff bedeuten.

2. Verfahren zur Herstellung der Verbindungen gemäß Anspruch 1 dadurch gekennzeichnet, daß man ein Phenylalkylhalogenid der Formel

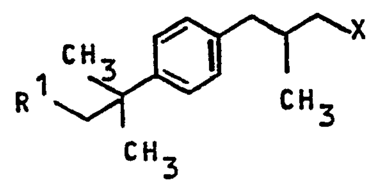

(2)

in der $R^1$ die in Anspruche 1 angegebene Bedeutung hat und X für Chlor oder Brom steht, oder einen entsprechenden Aldehyd der Formel

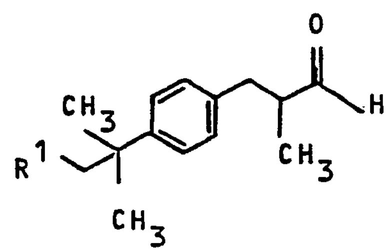

(3)

in der $R^1$ die in Anspruch 1 angegebenen Bedeutungen hat, umsetzt mit einem Piperidin der Formel

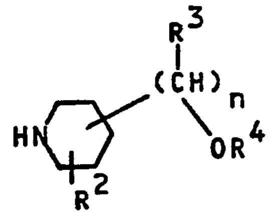

(4)

in der $R^2$, $R^3$, $R^4$ und n die in Anspruch 1 genannten Bedeutungen haben, wobei die Umsetzung des Aldehyds unter reduzierenden Bedingungen erfolgt.

3. Verfahren zur Herstellung der Verbindungen gemäß Anspruch 1 dadurch gekennzeichnet, daß man ein Hydroxypiperidin der Formel

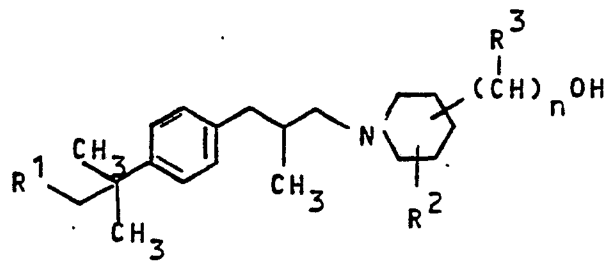

(5)

in der $R^1$, $R^2$, $R^3$ und n die in Anspruch 1 genannten Bedeutungen haben, mit einem Alkylierungsmittel der Formel

$R^{4'}Y$ (6)

in der $R^{4'}$, Alkyl, Alkenyl, Alkinyl, Halogenalkyl, Halogenalkenyl oder gegebenenfalls durch Halogen, $C_1$-$C_4$-Alkyl oder Trifluormethyl substituiertes Benzyl oder alpha- oder beta- Naphthylmethyl und Y Cl, Br, J bedeuten, oder

mit einem Acylchlorid der Formeln

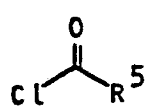

(7)

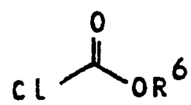

(8)

oder mit einem Isocyanat oder Carbamoylchlorid der Formel

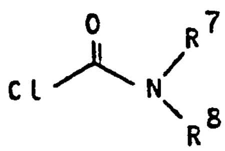

(9)

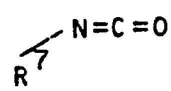

(10)

in denen $R^5$, $R^6$, $R^7$ und $R^8$ die in Anspruch 1 genannten Bedeutungen außer Wasserstoff haben, umsetzt.

4. Fungizides Mittel, enthaltend eine Verbindung gemäß Anspruch 1.

5. Fungizides Mittel, enthaltend ein Piperidin der Formel

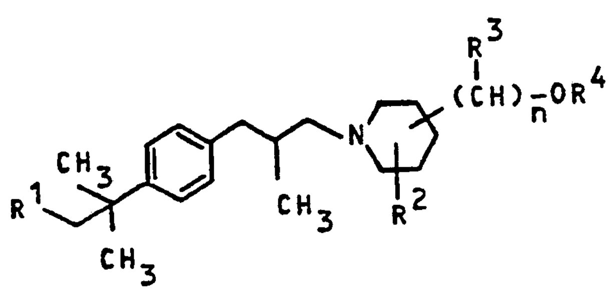

(1)

in der

**$R^1$ Wasserstoff, Alkyl, Halogen,**

**$R^2$, $R^3$ Wasserstoff, Alkyl,**

**$R^4$ Wasserstoff, Alkyl, Alkenyl, Alkinyl, Halogenalkyl, Halogenalkenyl, gegebenenfalls durch Halogen, $C_1$-$C_4$-Alkyl oder Trifluormethyl substituiertes Benzyl oder alpha- oder beta-Naphthylmethyl, $COR^5$, $CO_2R^6$, $CONR^7R^8$,**

**$R^5$ und $R^6$ Alkyl, gegebenenfalls durch Halogen oder $C_1$-$C_4$-Alkyl substituiertes Phenyl, gegebenenfalls durch Halogen substituiertes Benzyl,**

$R^7$ und $R^8$ Wasserstoff, Alkyl, gegebenenfalls durch Halogen substituiertes Phenyl, gegebenenfalls durch

Halogen substituiertes Benzyl, und

n die Zahlen 0, 1, 2 und 3 bedeuten, und seine für Pflanzen verträglichen Säureadditionssalze mit der Maßgabe, daß nicht gleichzeitig $R^4$ Wasserstoff, Acetyl oder Propionyl, n 0 oder 1 und $R^1$, $R^2$ und $R^3$ Wasserstoff bedeuten, und einen festen oder flüssigen Trägerstoff.

6. Verfahren zur Herstellung von Fungiziden, dadurch gekennzeichnet, daß man eine Verbindung der Formel 1 gemäß Anspruch 1 mit einem festen oder flüssigen Trägerstoff mischt.

7. Verfahren zur Bekämpfung von Fungi, dadurch gekennzeichnet, daß man ein Piperidin der Formel

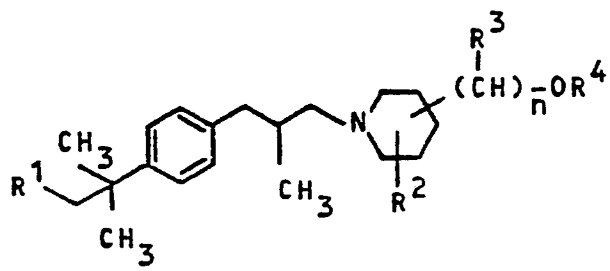

(1)

in der

$R^1$ Wasserstoff, Alkyl, Halogen,

$R^2$, $R^3$ Wasserstoff, Alkyl,

$R^4$ Wasserstoff, Alkyl, Alkenyl, Alkinyl, Halogenalkyl, Halogenalkenyl, gegebenenfalls durch Halogen, $C_1$-$C_4$-Alkyl oder Trifluormethyl substituiertes Benzyl oder alpha- oder beta-Naphthylmethyl, $COR^5$, $CO_2R^6$, $CONR^7R^8$,

$R^5$ und $R^6$ Alkyl, gegebenenfalls durch Halogen oder $C_1$-$C_4$-Alkyl substituiertes Phenyl, gegebenenfalls durch Halogen substituiertes Benzyl,

$R^7$ und $R^8$ Wasserstoff, Alkyl, gegebenenfalls durch Halogen substituiertes Phenyl, gegebenenfalls durch Halogen substituiertes Benzyl, und

n die Zahlen 0, 1, 2 und 3 bedeuten, und seine für Pflanzen verträglichen Säureadditionssalze mit der Maßgabe, daß nicht gleichzeitig $R^4$ Wasserstoff, Acetyl oder Propionyl, n 0 oder 1 und $R^1$, $R^2$ und $R^3$ Wasserstoff bedeuten,

auf Pilze oder durch Pilzbefall bedrohte Materialien, Flächen, Pflanzen oder Saatgüter einwirken läßt.

**Claims**

1. A piperidine of the formula

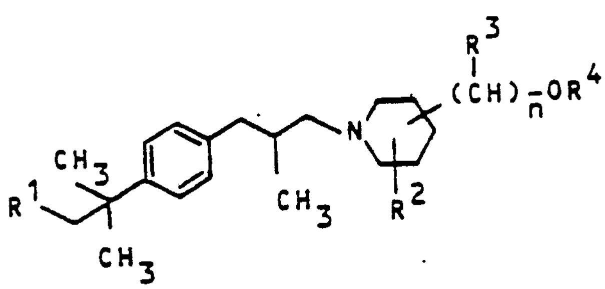

(1)

where

$R^1$ is hydrogen, alkyl or halogen,

$R^2$ and $R^3$ are hydrogen and alkyl,

$R^4$ is hydrogen, alkyl, alkenyl, alkynyl, haloalkyl, haloalkenyl, alpha- or beta-naphthylmethyl or benzyl which is unsubstituted or substituted by halogen, $C_1$-$C_4$-alkyl or trifluoromethyl, or $COR^5$, $CO_2R^6$ or $CONR^7R^8$,

$R^5$ and $R^6$ are alkyl, phenyl which is unsubstituted or substituted by halogen or $C_1$-$C_4$-alkyl, or unsubstituted or halogen-substituted benzyl,

$R^7$ and $R^8$ are hydrogen, alkyl, unsubstituted or halogen-substituted phenyl or unsubstituted or halogen-substituted benzyl, and

n is 0, 1, 2 or 3,

or a plant-tolerated acid addition salt thereof, with the proviso that, when $R^4$ is hydrogen, acetyl or

propionyl, n is not 0 or 1 and $R^1$, $R^2$ and $R^3$ are not hydrogen.

2. A process for the preparation of a compound as claimed in claim 1, wherein a phenylalkyl halide of the formula

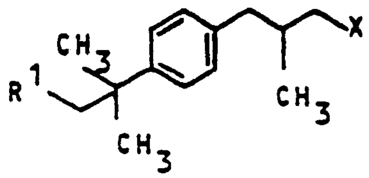

(2)

where $R^1$ has the meanings stated in claim 1 and X is chlorine or bromine, or a corresponding aldehyde of the formula

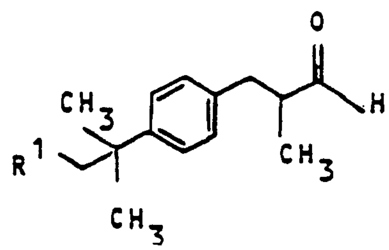

(3)

where $R^1$ has the meanings stated in claim 1, is reacted with a piperidine of the formula

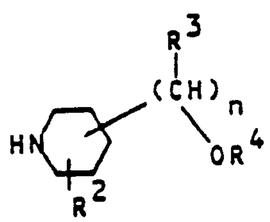

(4)

where $R^2$, $R^3$, $R^4$ and n have the meanings stated in claim 1, the reaction of the aldehyde being carried out under reduced conditions.

3. A process for the preparation of a compound as claimed in claim 1, wherein a hydroxypiperidine of the formula

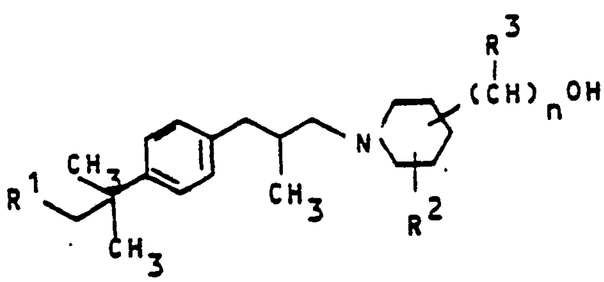

(5)

where $R^1$, $R^2$, $R^3$ and n have the meanings stated in claim 1, is reacted with an alkylating agent of the formula

$R^{4'}Y$ (6)

where $R^{4'}$ is alkyl, alkenyl, alkynyl, haloalkyl, haloalkenyl or alpha- or beta-naphthylmethyl or benzyl which is unsubstituted or substituted by halogen, $C_1$-$C_4$-alkyl or trifluoromethyl and Y is Cl, Br or I, or with an acyl chloride of the formulae.

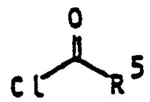

(7)

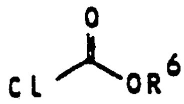

(8)

or with an isocyanate or carbamyl chloride of the formula

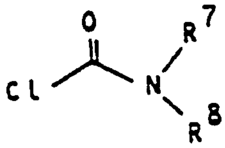

(9)

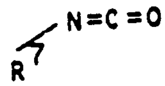

(10)

where $R^5$, $R^6$, $R^7$ and $R^8$ have the meanings stated in claim 1.

4. A fungicide containing a compound as claimed in claim 1.

5. A fungicide containing a piperidine of the formula

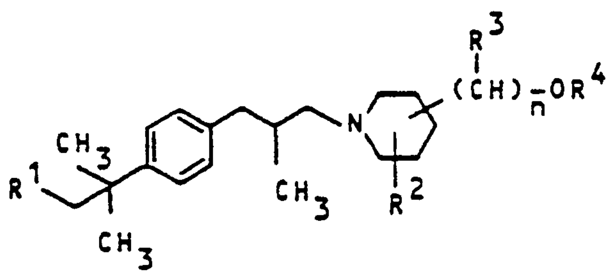

(1)

where $R^1$ is hydrogen, alkyl or halogen,

$R^2$ and $R^3$ are hydrogen and alkyl,

$R^4$ is hydrogen, alkyl, alkenyl, alkynyl, haloalkyl, haloalkenyl, alpha- or beta-naphthylmethyl or benzyl which is unsubstituted or substituted by halogen, $C_1$-$C_4$-alkyl or trifluoromethyl, or $COR^5$, $CO_2R^6$ or $CONR^7R^8$,

$R^5$ and $R^6$ are alkyl, phenyl which is unsubstituted or substituted by halogen or $C_1$-$C_4$-alkyl, or unsubstituted or halogen-substituted benzyl, $R^7$ and $R^8$ are hydrogen, alkyl, unsubstituted or halogen-substituted phenyl or unsubstituted or halogen-substituted benzyl, and

n is 0, 1, 2 or 3,

or a plant-tolerated acid addition salt thereof, with the proviso that, when $R^4$ is hydrogen, acetyl or propionyl, n is not 0 or 1 and $R^1$, $R^2$ and $R^3$ are not hydrogen, and a solid or liquid carrier.

6. A process for the preparation of a fungicide, wherein a compound of the formula I as claimed in claim 1 is mixed with a solid or liquid carrier.

7. A method of controlling fungi, wherein a piperidine of the formula

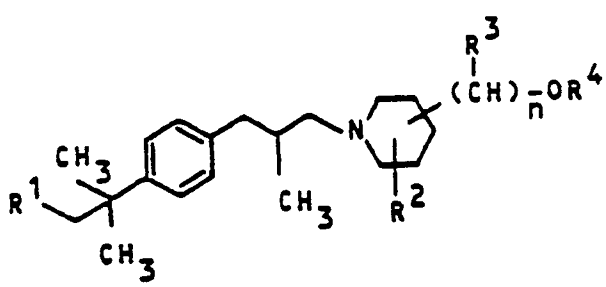

(1)

where $R^1$ is hydrogen, alkyl or halogen,

$R^2$ and $R^3$ are hydrogen and alkyl,

$R^4$ is hydrogen, alkyl, alkenyl, alkynyl, haloalkyl, haloalkenyl, alpha- or beta-naphthylmethyl or benzyl which is unsubstituted or substituted by halogen, $C_1$-$C_4$-alkyl or trifluoromethyl, or $COR^5$, $CO_2R^6$ or $CONR^7R^8$,

$R^5$ and $R^6$ are alkyl, phenyl which is unsubstituted or substituted by halogen or $C_1$-$C_4$-alkyl, or unsubstituted or halogen-substituted benzyl,

$R^7$ and $R^8$ are hydrogen, alkyl, unsubstituted or halogen-substituted phenyl or unsubstituted or halogen-substituted benzyl, and

n is 0, 1, 2 or 3,

or a plant-tolerated acid addition salt thereof, with the proviso that, when $R^4$ is hydrogen, acetyl or propionyl, n is not 0 or 1 and $R^1$, $R^2$ and $R^3$ are not hydrogen,

is allowed to act on fungi or on materials, areas, plants or seed threatened by fungal attack.

**Revendications**

1. Pipéridine de formule

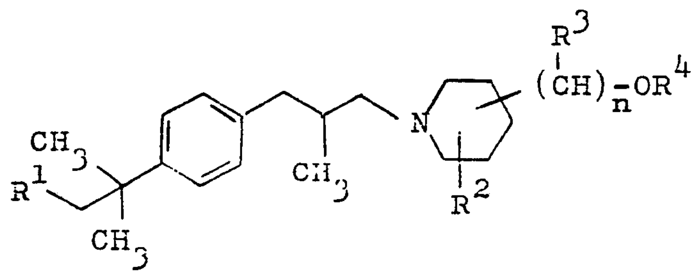

(1)

dans laquelle

$R^1$ représente hydrogène, alkyle, halogène,

$R^2$, $R^3$ hydrogène, alkyle,

$R^4$ hydrogène, alkyle, alcényle, alcinyle, halogénalkyle, halogénalcényle, benzile ou α- ou β-naphtylméthyle éventuellement substitué par halogène, alkyle en $C_1$-$C_4$ ou trifluorométhyle, $COR^5$, $CO_2R^6$, $CONR^7R^8$,

$R^5$ et $R^6$ alkyle, phényle éventuellement substitué par halogène ou alkyle en $C_1$-$C_4$, benzyle éventuellement substitué par halogène,

$R^7$ et $R^8$ hydrogène, alkyle, phényle éventuellement substitué par halogène, benzyle éventuellement substitué par halogène, et

n représente les nombres 0, 1, 2 et 3 et ses sels d'addition d'acide compatibles pour les plantes, sous réserve que, ne représentent pas simultanément $R^4$, hydrogène, acétyle ou propionyle, n, 0 ou 1 et $R^1$, $R^2$ et $R^3$, hydrogène.

2. Procédé de préparation des composés selon la revendication 1, caractérisé par le fait que l'on fait réagir un halogénure de phénylalkyle de formule

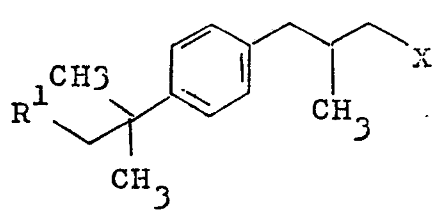

(2)

dans laquelle $R^1$ a les significations données dans la revendication 1 et X est mis pour chlore ou brome, ou un aldéhyde correspondant de formule

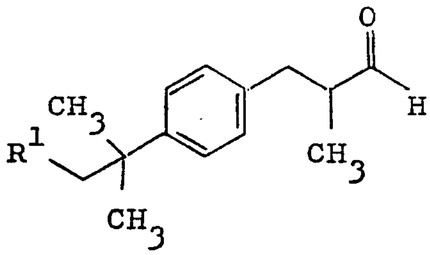

(3)

dans laquelle $R^1$ a les significations données dans la revendication 1, avec une pipéridine de formule

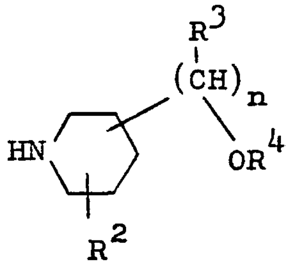

(4)

dans laquelle $R^2$, $R^3$, $R^4$ et n ont les significations données dans la revendication 1, la réaction de l'aldéhyde se déroulant dans des conditions réductrices.

3. Procédé de préparation des composés selon la revendication 1, caractérisé par le fait que l'on fait réagir une hydroxypipéridine de formule

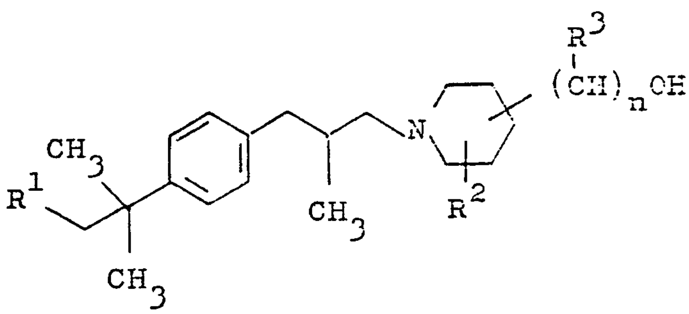

(5)

dans laquelle $R^1$, $R^2$, $R^3$ et n on les significations données dans la revendication 1, avec un agent d'alkylation de formule

$R^{4'}Y$ (6)

dans laquelle $R^{4'}$ représente alkyle, alcényle, alcinyle, halogénalkyle, halogénalcényle, ou benzyle ou α- ou β-naphtylméthyle éventuellement substitués par halogène, alkyle en $C_1$-$C_4$ ou trifluorométhyle et 1 représente Cl, Br, I, ou avec un chlorure d'acyle des formules

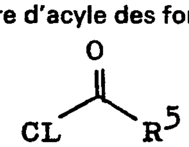

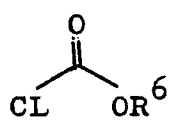

(7) (8)

ou avec un isocyanate ou un chlorure de carbamoyle de formule

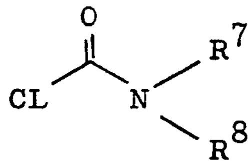

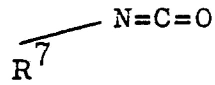

(9) (10)

dans lesquelles $R^5$, $R^6$, $R^7$ et $R^8$ ont les significations données dans la revendication 1.

4. Agent fongicide, contenant un composé selon la revendication 1.

5. Agent fongicide contenant une pipéridine de formule

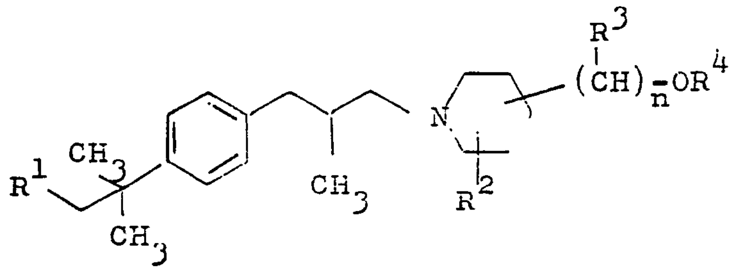

(1)

dans laquelle

$R^1$ représente hydrogène, alkyle, halogène,

$R^2$, $R^3$ hydrogène, alkyle,

$R^4$ hydrogène, alkyle, alcényle, alcinyle, halogénalkyle, halogénalcényle, benzile ou α- ou β-naphtylméthyle éventuellement substitué par halogène, alkyle en $C_1$-$C_4$ ou trifluorométhyle, $COR^5$, $CO_2R^6$, $CONR^7R^8$,

$R^5$ et $R^6$ alkyle, phényle éventuellement substitué par halogène ou alkyle en $C_1$-$C_4$, benzyle éventuellement substitué par halogène,

$R^7$ et $R^8$ hydrogène, alkyle, phényle éventuellement substitué par halogène, benzyle éventuellement substitué par halogène, et

n représente les nombres 0, 1, 2 et 3 et ses sels d'addition d'acide compatibles pour les plantes, sous réserve que, ne représentant pas simultanément $R^4$, hydrogène, acétyle ou propionyle, n, 0 ou 1 et $R^1$, $R^2$ et $R^3$, hydrogène et un support solide ou liquide.

6. Procédé de préparation de fongicides, caractérisé par le fait que l'on mélange un composé de formule I selon la revendication 1 avec un support solide ou liquide.

7. Procédé pour lutter contre les champignons, caractérisé par le fait que l'on fait agir sur le champignons ou les matériaux, surfaces, plantes ou semences menacés d'une attaque par les champignons, une pipéridine de formule

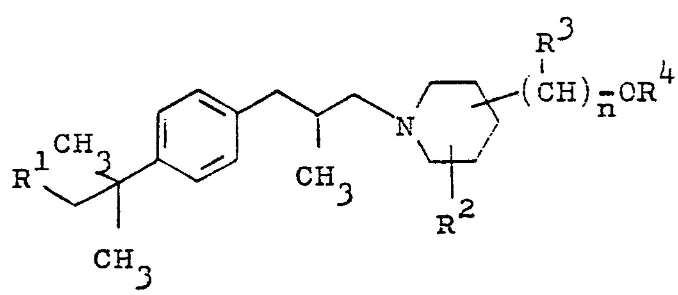

(1)

dans laquelle

$R^1$ représente hydrogène, alkyle, halogène,

$R^2$, $R^3$ hydrogène, alkyle,

$R^4$ hydrogène, alkyle, alcényle, alcinyle, halogénalkyle, halogénalcényle, benzile ou α- ou β-naphtylméthyle éventuellement substitué par halogène, alkyle en $C_1$-$C_4$ ou trifluorométhyle, $COR^5$, $CO_2R^6$, $CONR^7R^8$,

$R^5$ et $R^6$ alkyle, phényle éventuellement substitue par halogène ou alkyle en $C_1$-$C_4$, benzyle éventuellement substitué par halogène,

$R^7$ et $R^8$ hydrogène, alkyle, phényle éventuellement substitué par halogène, benzyle éventuellement substitué par halogène, et

n représente les nombres 0, 1, 2 et 3 et ses sels d'addition d'acide compatibles pour les plantes, sous réserve que, ne représentent pas simultanément $R^4$, hydrogène, acétyle ou propionyle, n, 0 ou 1 et $R^1$, $R^2$ et $R^3$, hydrogène.